(19) Europäisches Patentamt
European Patent Office
Office européen des brevets

(11) Veröffentlichungsnummer : **0 129 105**
**B1**

(12) **EUROPÄISCHE PATENTSCHRIFT**

(45) Veröffentlichungstag der Patentschrift : **21.01.87**

(51) Int. Cl.[4] : **C 08 G 59/16**, C 09 D 5/44, C 07 D263/04, C 08 K 5/35

(21) Anmeldenummer : **84106052.8**

(22) Anmeldetag : **28.05.84**

(54) **Verfahren zur Herstellung von Härterkomponenten für Lackbindemittel.**

(30) Priorität : **20.06.83 AT 2258/83**

(43) Veröffentlichungstag der Anmeldung : **27.12.84 Patentblatt 84/52**

(45) Bekanntmachung des Hinweises auf die Patenterteilung : **21.01.87 Patentblatt 87/04**

(84) Benannte Vertragsstaaten : **DE FR GB IT**

(56) Entgegenhaltungen : **EP-A- 0 028 402**

(73) Patentinhaber : **Vianova Kunstharz Aktiengesellschaft**
**A-8402 Werndorf (AT)**

(72) Erfinder : **Paar, Willibald, Dr.**
**Haberlandtweg 23**
**A-8045 Graz (AT)**
Erfinder : **Schipfer, Rudolf, Dr.**
**Ernst Haeckelstrasse 53**
**A-8010 Graz (AT)**

(74) Vertreter : **Pitter, Robert, Dr. et al**
**Postfach 191 Leechgasse 21**
**A-8011 Graz (AT)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 129 105 B1

Jouve, 18, rue St-Denis, 75001 Paris, France

**Beschreibung**

Die Erfindung betrifft ein Verfahren zur Herstellung verbesserter Härterkomponenten für Lackbindemittel, welche aufgrund ihres Aufbaues durch Umesterung vernetzt werden können.

In den Europäischen Patent EP-A1-12 463 werden wärmehärtende Bindemittelzusammensetzungen beschrieben, bei welchen die Vernetzung beim Einbrennen durch Umesterung zwischen den Hydroxylgruppen einer Harzkomponente, welche frei von Säuregruppen und äthylenisch ungesättigten Gruppierungen ist, und einem säuregruppenfreien Polyester mit mehr als einer β-Hydroxygruppe in Gegenwart eines Umesterungskatalysators erfolgt. Soferne die Harzkomponente ein kationisches wasserverdünnbares Harz ist, kann das Bindemittelsystem auch durch Elektrotauch-Lackierung (ETL) appliziert werden.

Die Einführung der für Umesterungsreaktionen besonders geeigneten β-Hydroxyestergruppierung erfolgt gemäß dieser Literaturstelle durch Umsetzung eines Polycarbonsäureanhydrids mit Glykolen, Glykolmonoäthern, Polyolen und/oder vorzugsweise mit Monoepoxiden. Bevorzugt werden als β-Hydroxygruppen tragende Polyester solche aus Trimellithsäureanhydrid und einem Glycidylester von gesättigten aliphatischen Carbonsäuren mit 9 bis 11 C-Atomen, deren Carboxylgruppe an ein tertiäres Kohlenwasserstoffatom gebunden ist (in der Literaturstelle als « Glycidylester C 10 E » bezeichnet), eingesetzt. Bei der Vernetzung dieser Bindemittel werden die als β-Hydroxyester gebundenen Glykole bei Einbrenntemperaturen von 150 bis 200 °C abgespalten und aus dem Überzug entfernt. Abgesehen von den — auch bei Verwendung von Umesterungskatalysatoren — notwendigen relativ hohen Einbrenntemperaturen stellt der hohe Anteil an Spaltprodukten einen wesentlichen Nachteil dieser Vernetzungskomponente dar. Überdies müssen dabei hochwertige Lackrohstoffe abgespalten und aus dem Lackfilm entfernt werden, was nicht nur aus wirtschaftlichen sondern auch aus ökologischen Gründen unvorteilhaft ist.

In der AT-B-372099 (Priorität A 5083/81) werden Vernetzungskomponenten vorgeschlagen, deren Carboxylgruppen mit niederen Alkoholen verestert sind. Die Carboxylgruppen stammen dabei von speziellen Dicarbonsäuren, insbesonders der Malonsäure. Diese Dicarbonsäureester können auch Endglieder von oligomeren oder polymeren Estern darstellen.

Es wurde nun gefunden, daß Härterkomponenten, welche neben der Malonesterstruktur eine basische Funktion in Form einer Oxazolidingruppe aufweisen, besonders günstige Eigenschaften bei der Verarbeitung in kathodischen ET-Lacken aufweisen. Aufgrund ihres Aufbaues weisen diese Härterkomponenten neben den funktionellen Esterstrukturen keine verseifbaren Estergruppen auf.

Die vorliegende Erfindung betrifft demgemäß ein Verfahren zur Herstellung von Vernetzungskomponenten für durch Umesterung vernetzbare, wasserverdünnbare, kationische Lackbindemittel, welches dadurch gekennzeichnet ist, daß man

(A) ein Knoevenagel-Reaktionsprodukt aus einer Carbonylverbindung und einer Verbindung der Formel

$$X-CH_2-Y$$

wobei
X —COOR, —CN oder —$COCH_3$,
Y —COOR ist und
R einen Alkylrest mit 1-4 C-Atomen darstellt

mit

(B) einem eine sekundäre Aminogruppen aufweisenden substituierten Oxazolidin der allgemeinen Formel

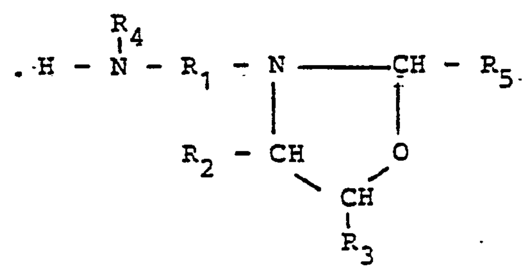

wobei
$R_1$ ein geradkettiger oder verzweigter oder cyclischer Alkylenrest mit 2 bis 12 C-Atomen oder ein Aralkylenrest,
$R_2$ ein Wasserstoffatom oder eine Methylgruppe,
$R_3$ ein Wasserstoffatom oder ein Alkylrest,
$R_4$ ein nach Reaktion mit einem aktiven Wasserstoffatom verbleibender Rest eines in bezug auf die

Doppelbindungen monofunktionellen Acryl- oder Methacrylmonomeren und

$R_5$ ein Wasserstoffatom oder ein Alkylrest mit 1 bis 12 C-Atomen oder ein gegebenenfalls substituierter Arylrest ist,

im Sinne einer Michael-Addition reagiert und das Reaktionsprodukt über die aktiven Methylengruppen bei 30 bis 35 °C mit

(C) einem Di- oder Polyisocyanat umsetzt, wobei die Menge an isocyanatgruppen den aktiven Methylengruppen der Oxazolidinringe entspricht.

Die Erfindung betrifft weiters die Verwendung solcher Vernetzungskomponenten zur Vernetzung von hydroxy- und aminofunktionellen Basisharzen und die Verwendung solcher Harz/Härtersysteme als Bindemittel für die kathodische Abscheidung nach dem ETL-Verfahren.

Für das erfindungsgemäße Verfahren kann beispielsweise folgender Reaktionsablauf angenommen werden :

(A) Knoevenagel-Reaktion :

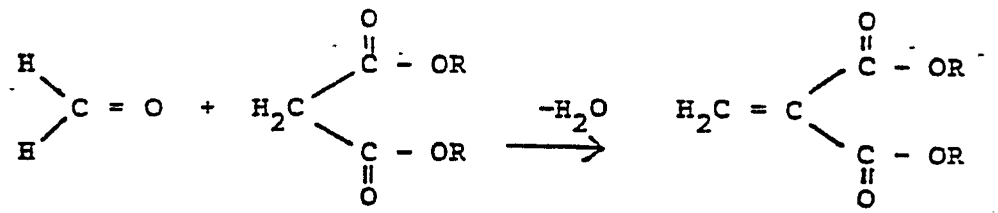


Komponente (A)

(B) Michael-Addition

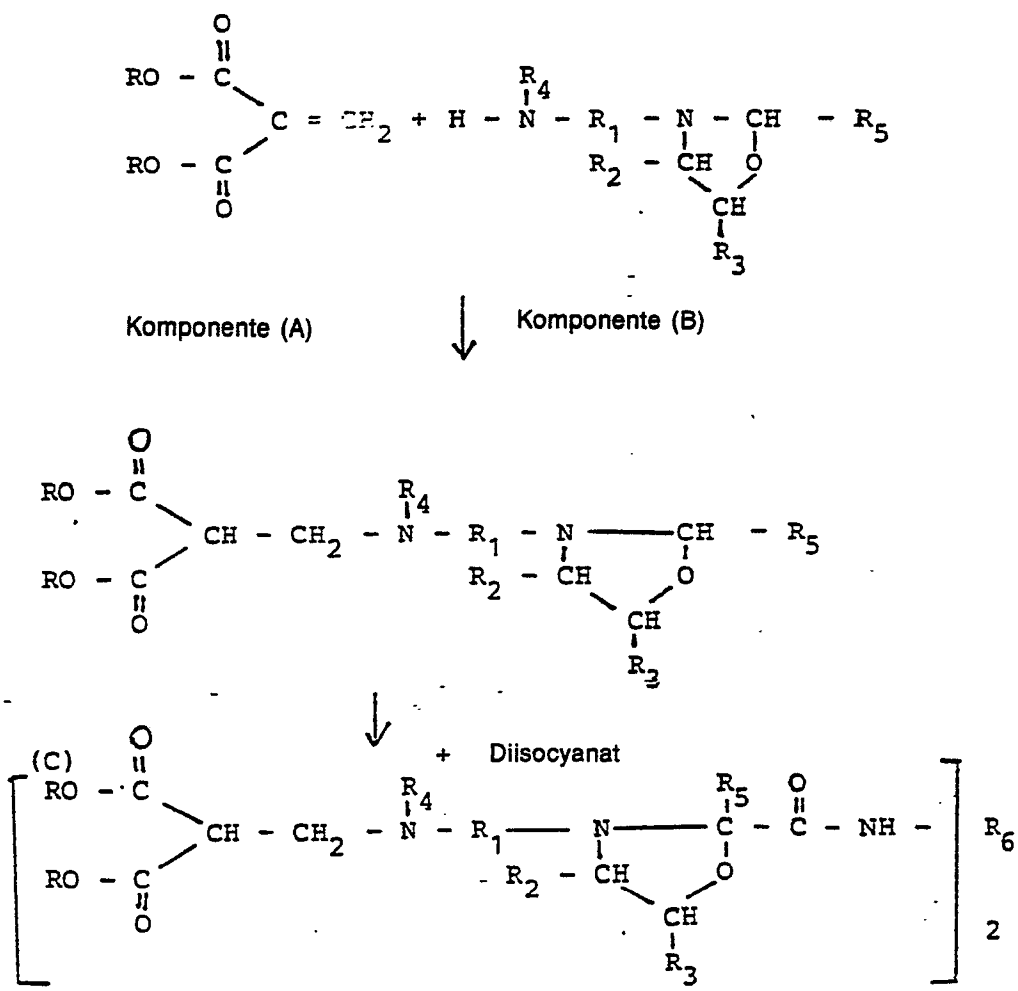

$R_6$ = ein aromatischer, aliphatischer oder cycloaliphatischer Kohlenwasserstoffrest.

Wie sich auch aus der Formel für die erfindungsgemäß hergestellten Vernetzungskomponenten ergibt, zeigen diese einige wesentliche Verbesserungen gegenüber den Produkten des Standes der Technik.

(a) Das System weist außer den funktionellen Estergruppen keine Gruppierungen auf, durch deren Spaltung eine wesentliche Verkleinerung des Moleküles erfolgen könnte.

(b) Durch die Anwesenheit basischer Gruppen ist eine gleiche ionische Gruppierung, wie im kationischen Basisharz gegeben, sodaß bei der kathodischen Abscheidung eine äquivalente Abscheidung gewährleistet ist.

(c) Durch die Auswahlmöglichkeit bei den Resten $R_1$ und $R_4$ sind weitgehende Möglichkeiten für eine Plastifizierung des Härtermoleküls und damit des Gesamtsystems gegeben. Außer der Oberflächenqualität der abgeschiedenen Filme kann dadurch auch die Flexibilität der eingebrannten Überzüge beeinflußt werden.

(d) Durch das Vorhandensein von Säureamidgruppen wird die Haftfestigkeit der Filme wesentlich verbessert.

(e) Durch die Oxazolidingruppen wird sowohl die Pigmentbenetzung der Bindemittelsysteme als auch die Emulgierung der Harzkomponente und anderer Zusätze verbessert.

In der Stufe (A) wird ein Aldehyd oder ein Keton mit einem CH— aciden Ester im Sinne einer Knoevenagel-Reaktion umgesetzt. Die unter diesem Namen bekannte Reaktion zwischen Methylen-Komponenten mit hoher C—H-Acidität und Carbonylverbindungen ist z. B. in dem Buch « Organikum », 12. Auflage, VEB Deutscher Verlag der Wissenschaften Berlin 1973, auf Seite 508 ff, beschrieben. (s. auch Römpp's Chemie Lexikon, 7. Auflage).

Als Ausgangsmaterialien werden bevorzugt die Diester der Malonsäure mit $C_1$-$C_4$ Alkanolen, wie Dimethylmalonat, Diäthylmalonat, n- oder iso-Propylmalonat-, n-, iso- oder tert.Butylmalonat, Diäthylmethylmalonat eingesetzt. In gleicher Weise können auch die Alkyl- oder Cycloalkylester der Cyanessigsäure, wie Cyanessigsäuremethyl-, äthyl-, propyl-, butyl-, cyclopentyl-, -cyclohexylester oder der Acetessigsäuremethyl- oder äthylester eingesetzt werden.

Als Aldehyd wird vorzugsweise Formaldehyd, und zwar insbesonders in seiner polymerisierten Form (Paraformaldehyd) eingesetzt. Die Verwendung höherer Aldehyde wie Acetaldehyd oder eines Butanals oder eines aromatischen Aldehyds ist zwar möglich, bringt jedoch keine wesentlichen Vorteile. Weiters können auch Ketone, z. B. das Methylisobutylketon zum Einsatz kommen.

Die Herstellung der Komponente (A) erfolgt nach der Variante nach COPE (siehe obengenannte Literaturstelle). Man arbeitet dabei vorteilhaft in der Weise, daß der Paraformaldehyd bei 60-70 °C portionsweise dem Ester zugegeben und in diesem gelöst wird. Gegebenenfalls kann als Katalysator für die Reaktion dem Ester eine Mischung aus Piperidin und Ameisensäure in einer Menge von etwa 1 Mol-% zugesetzt werden. Die Reaktionstemperatur soll bis zur vollständigen Auflösung des Paraformaldehyds 90 °C nicht überschreiten. Anschließend wird die Temperatur langsam erhöht und das entstehende Reaktionswasser bei 120-150 °C durch azeotrope Destillation mit einem Schleppmittel, z. B. einem aliphatischen oder einem aromatischen Kohlenwasserstoff, aus dem Reaktionsgemisch entfernt. Die Reaktion wird anhand des gebildeten Reaktionswassers verfolgt.

Das In Stufe (B) eingesetzte eine sekundäre Aminogruppe tragende substituierte Oxazolidin der allgemeinen Formel

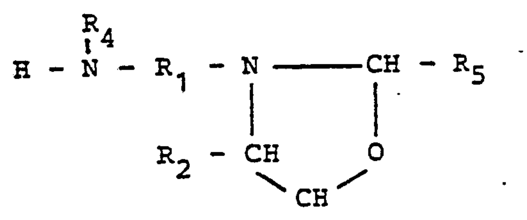

wobei die Reste $R_1$ bis $R_5$ die oben angegebene Bedeutung haben, wird durch Umsetzung eines Alkylamino-β-hydroxyamins mit einer in bezug auf die Doppelbindung monofunktionellen Acryl- oder Methacrylverbindung und nachfolgendem Ringschluß, z. B. mit Formaldehyd gemäß folgendem Formelschema erhalten :

(Siehe Formel Seite 5 f.)

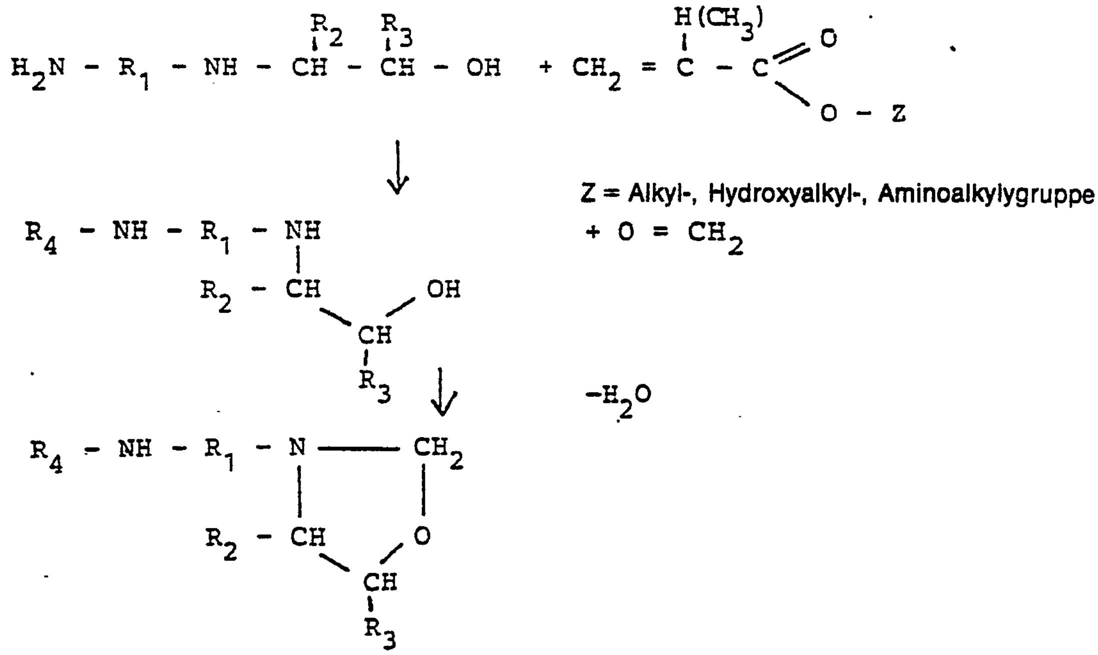


Als Alkylamino-β-hydroxyamine werden vorzugsweise handelsübliche Produkte, wie Aminoäthyläthanolamin ($NH_2$—$(CH_2)_2$—NH—$(CH_2)_2$—OH) oder das Aminopropyläthanolamin oder das Aminoäthylisopropanolamin eingesetzt.

Als Acryl- oder Methacrylmonomere können neben den homologen Estern der Acrylsäure bzw. der Methacrylsäure mit Monoalkoholen auch tertiäre Aminogruppen tragende Monomere, beispielsweise Dimethylaminoäthylacrylat und dessen Homologe oder die homologen Hydroxyalkylacrylate eingesetzt werden.

Die Herstellung der erfindungsgemäß eingesetzten Oxazolidinamine der Formel (III) erfolgt in erster Stufe durch langsame Zugabe des Acrylmonomeren zum vorgelegten Amin unter Kühlung bei 30 bis 50 °C. Die Umsetzung wird anschließend bei 70 bis 90 °C während 1 bis 3 Stunden vervollständigt. Beim Einsatz von Methacrylmonomeren wird die Temperatur in dieser Phase vorteilhaft auf etwa 140 °C gesteigert. Der Ringschluß mit der Carbonylverbindung erfolgt bei 80 bis 115 °C, wobei das entstehende Reaktionswasser azeotrop mit Hilfe eines Schleppmittels, z. B. einem Benzinkohlenwasserstoff mit einem Siedebereich von 80 bis 120 °C, abgetrennt wird.

Die Umsetzung der Komponenten (A) und (B) erfolgt im Sinne einer Michael-Addition bei 50 bis 100 °C während 1 bis 3 Stunden. Die Reaktionsschritte (A) und (B) können auch gemeinsam erfolgen, wobei in diesem Fall auch auf den Katalysator für die Knoevenagel-Reaktion verzichtet werden kann.

In der Stufe (C) wird das Michael-Additionsprodukt aus (A) und (B) über die aktive Methylengruppe des Oxazolidinringes mit der Isocyanatgruppe eines Di- oder gegebenenfalls Polyisocyanates umgesetzt. Pro Isocyanatgruppe wird ein Mol des Zwischenproduktes aus (A) und (B) eingesetzt. Die Reaktion erfolgt so, daß das Isocyanat bei 35-45 °C dem Michael-Additionsprodukt in ca. 1 Stunde zugegeben wird. Dann wird bei dieser Temperatur die Reaktion bis zum vollständigen Verbrauch aller Isocyanatgruppen weitergeführt.

Als Basisharze zur Kombination mit den erfindungsgemäß hergestellten Vernetzungskomponenten können Produkte eingesetzt werden, welche über eine ausreichende Anzahl veresterungsfähiger bzw. amidierungsfähiger Gruppen verfügen um eine genügende Vernetzung des Lackfilms zu gewährleisten. Harze dieser Art sind in der EP-A1-12 463 oder in der AT-B-372099 in größerer Zahl beschrieben. Bevorzugt werden Harze auf der Basis von Epoxidharzen eingesetzt, wobei insbesonders für die kathodische Abscheidung Epoxidharz-Amin-Addukte, welche gegebenenfalls im Sinne einer inneren Flexibilisierung modifiziert sein können, in Frage kommen.

Die erfindungsgemäß hergestellten Härterkomponenten werden in einer Menge von 15 bis 40 Gew.-% eingesetzt. Die Menge richtet sich im Einzelfall im wesentlichen nach den im Basisharz verfügbaren funktionellen Gruppen, welche einer Umesterungs- oder Amidierungsreaktion mit den bei erhöhter Temperatur freigesetzten Carboxylgruppen zugänglich sind.

Die Verarbeitung der Bindemittelsysteme zu wasserverdünnbaren Überzugsmitteln, d. h. die Neutralisation mit Säure, die Verdünnung auf die Applikationsform oder die in den meisten Fällen erfolgende Einarbeitung von Pigmenten und Füllstoffen, sind dem Fachmann ebenso bekannt, wie die möglichen Applikationsformen. Ein bevorzugter Einsatzbereich der Überzugsmittel, welche die erfindungsgemäße Härterkomponente enthalten, ist das K-ETL-Verfahren, bei welchem das zu beschichtende Werkstück als Kathode geschaltet ist.

Die folgenden Beispiele sollen die Erfindung erklären, ohne daß ihr Umfang dadurch beschränkt wird. Alle Mengenangaben sind soweit nicht anders angegeben, Gewichtseinheiten und beziehen sich bei

den Harzkomponenten auf Festharz.

Folgende Abkürzungen werden in den Beispielen verwendet :

| | |
|---|---|
| DEA | Diäthylamin |
| DOLA | Diäthanolamin |
| DIPA | Diisopropanolamin |
| DEAPA | Diäthylaminopropylamin |
| DETA | Diäthylentriamin |
| AEEA | Aminoäthyläthanolamin |
| AEPA | Aminopropyläthanolamin |
| BUAC | Butylacrylat |
| EHA | 2-Äthylhexylacrylat |
| EMA | Äthylmethacrylat |
| MDE | Malonsäurediäthylester |
| ACE | Acetessigsäureäthylester |
| CEE | Cyanessigsäureäthylester |
| FA | Paraformaldehyd (91 %) |
| BZA | Benzaldehyd |
| SA | Salicylaldehyd |
| TDI | Toluylendiisocyanat (handelsübliches Isomerengemisch) |
| IPDI | Isophorondiisocyanat |
| HMDI | 1,6-Hexamethylendiisocyanat |
| HEOX | N-Hydroxyäthyloxazolidin |
| MGL | Äthylenglykolmonomethyläther |
| EGL | Monoäthylenglykolmonoäthyläther |
| DEGM | Diäthylenglykoldimethyläther |
| MIBK | Methylisobutylketon |
| PSA | o-Phthalsäureanhydrid |
| THPSA | Tetrahydrophthalsäureanhydrid |
| TOFS | Tallölfettsäure |
| INS | Isononansäure |
| FH | 2-Äthylhexanol |

Die in den Beispielen eingesetzten Basisharze (BH) werden in folgender Weise hergestellt.

(BH I) : In einem mit Rührer, Thermometer und Rückflußkühler ausgestattetem Reaktionsgefäß werden 1 000 g eines Expoxidharzes (Basis Bisphenol-A ; Epoxyäquivalentgewicht ca. 500) in 512 g EGL bei 60 bis 70 °C gelöst. Anschließend werden 37 g DEA und 158 g DOLA zugegeben und der Ansatz 3 Stunden bei 100 °C reagiert. Das Produkt hat eine OH-Zahl entsprechend 375 mg KOH/g.

(BH II) : 380 g eines Epoxidnovolakharzes (Epoxidäquivalent ca. 190) werden in 354 g DEGM gelöst und bei 80 °C mit 269 g eines Halbesters aus THPSA und HEOX, sowie mit 37 g DEA und 140 g TOFS bis zu einer Säurezahl von unter 3 mg KOH/g umgesetzt. Anschließend wird das Harz nach Zusatz von 10 mM Essigsäure (3 normal) pro 100 g Festharz 3 Stunden bei 65 °C gerührt. Das Produkt hat eine OH-Zahl entsprechend 270 mg KOH/g.

(BH III) : Zu einer Lösung von 1 000 g eines Epoxidharzes (Basis Bisphenol A ; EG ca. 500) in 551 g MIBK werden bei 70 °C 168 g INS, 53 g DOLA und 33 g DEAPA zugegeben und das Reaktionsgemisch bei 95 bis 100 °C solange gehalten, bis eine Säurezahl von unter 3 mg KOH/g erreicht ist (Hydroxylzahl = 270 mg KOH/g).

(BH IV) : 400 g eines Epoxidharzes (Bisphenol A ; EG 200) werden in 261 g MGL gelöst und mit 278 g eines Halbesters aus PSA und EH, 67 g DIPA und 37 g DEA bei 90 bis 95 °C umgesetzt, bis eine Säurezahl von weniger als 3 mg KOH/g erreicht ist (Hydroxylzahl = 230 mg KOH/g).

(BH V) : 1 000 g Epoxidharz (Bisphenol A ; EG ca. 500) werden in 663 g MIBK gelöst und bei 60 °C mit 267 g eines Diketimins aus 1 Mol DETA und 2 Mol MIBK, sowie 280 g TOFS versetzt und bei 75 bis 80 °C bis zu einer Säurezahl von weniger als 3 mg KOH/g reagiert (Hydroxylzahl 145 mg KOH/g).

Die Oxazolidinverbindungen (Komponente B) werden in der Weise hergestellt, daß man unter Kühlen bei 30 bis 35 °C das Acrylat innerhalb einer Stunde dem vorgelegten Amin zugibt und die Reaktion während 1 bis 3 Stunden bei 70 bis 90 °C (beim Einsatz von Methacrylaten bei ca. 140 °C) vervollständigt. Anschließend wird bei 70 bis 80 °C die Carbonylverbindung und das Kreislaufmittel (Spezialbenzin mit Siedebereich 80 bis 120 °C oder ein ähnlicher Benzinkohlenwasserstoff) zugegeben und die Umsetzung bei 80 bis 115 °C unter azeotroper Entfernung des Reaktionswassers durchgeführt. Anschließend wird das Kreislaufmittel im Vacuum abgezogen.

Die Komponente (A) wird durch Reaktion des C—H-aciden Esters mit der Carbonylverbindung bei 80 bis 150 °C hergestellt, wobei das Reaktionswasser durch ein Schleppmittel, wie das obengenannte Spezialbenzin, entfernt wird.

Erfindungsgemäße Beispiele 1-6 :

In der Tabelle 1 sind die Mengenangaben und Reaktionsbedingungen für die Herstellung der Ausgangskomponenten und die erfindungsgemäßen Härterkomponenten zusammengefaßt. Vorteilhafterweise wird in der ersten Stufe die Oxazolidinverbindung (Komponente B) hergestellt, an welche dann bei 50 bis 100 °C innerhalb von 1 bis 3 Stunden die Komponente (A) addiert wird.

Die Knoevenagel-Kondensation und die Michael-Addition kann auch in einer gemeinsamen Reaktion erfolgen. Dabei wird zur Oxazolidinverbindung der C—H-acide Ester und die Carbonylverbindung zugegeben. Die Reaktion erfolgt bei 80 bis 130 °C bei gleichzeitiger azeotroper Entfernung des Reaktionswassers.

Das Reaktionsprodukt wird anschließend mit dem Di- oder Polyisocyanat umgesetzt, wobei die Menge der Isocyanatgruppen den verfügbaren Methylengruppen im Oxazolidinring äquivalent ist. Die Reaktion erfolgt, gegebenenfalls in Gegenwart eines inerten Lösungsmittels (Xylol, MIBK) bei 35 bis 45 °C, wobei vorteilhaft das Isocyanat während 45 bis 90 Minuten zugegeben wird. Die Reaktion ist beendet, wenn der Isocyanatwert auf praktisch Null gesunken ist.

Die Verwendung der gemäß Beispiel 1 bis 6 hergestellten Härterkomponenten in Kombination mit Basisharzen als Überzugsmittel erfolgt durch gründliches Vermischen der Komponenten und anschließender Neutralisation mit Säuren, vorzugsweise Ameisensäure bis die gewünschte Verdünnbarkeit mit Wasser gegeben ist. Vor dem Verdünnen mit deionisiertem Wasser, werden gegebenenfalls die Pigmente und Füllstoffe und/oder Katalysatoren in der Harzmischung dispergiert. Diese Arbeitsgänge können auch vor der Säurezugabe bzw. mit den Einzelkomponenten erfolgen.

Beispiele für solche Bindemittelsysteme auf Basis der in der oben beschriebenen Weise hergestellten Produkte, sind in der Tabelle 2 zusammengefaßt.

(Siehe Tabelle 1 Seite 8 f.)

Tabelle 1

| | KOMPONENTE (A) | | | KOMPONENTE (B) | | | | KOMPONENTE (C) | |
|---|---|---|---|---|---|---|---|---|---|
| BEI-SPIEL | C-H-acider ESTER | CARBONYL-VERBINDUNG | REAKTIONS-BEDINGGEN Std/°C | AMIN | ACRYL-MONOMERES | REAKTIONS-BEDINGUNGEN Std./°C | ALDEHYD | DIISOCYANAT | REAKTIONS-BEDINGUNGEN Std./°C |
| 1 | 320 MDE | 200 MIBK | 8/145 | 208 AEEA | 256 BUAC | 2 / 70 | 212 BZA | 222 IPDI | 2 / 45 |
| 2 | 320 MDE | 66 FA | 6/120 | 208 AEEA | 368 EHA | 2 / 70 | 66 FA | 174 TDI | 1 / 40 |
| 3 | 226 CEE | 196 CHX | 6/130 | 236 APEA | 228 EMA | 2 /140 | 212 BZA | 168 HMDI | 2 / 35 |
| 4 | 226 CEE | 66 FA | 7/125 | 208 AEEA | 256 BUAC | 2 / 70 | 244 SA | 174 TDI | 1 / 40 |
| 5 | 260 ACE | 200 MIBK | 9/140 | 236 APEA | 368 EHA | 2 / 75 | 66 FA | 222 IPDI | 2 / 45 |
| 6 | 260 ACE | 66 FA | 8/125 | 208 AEEA | 228 EMA | 2 /140 | 66 FA | 174 TDI | 1 / 40 |

Tabelle 2

| BASISHARZ | | HÄRTERKOMPONENTE | AMEISENSÄURE | pH-Wert |
|---|---|---|---|---|
| TEILE FESTHARZ | | TEILE FESTHARZ | MILLIMOL/1000 g | 10%ig Lg |
| 70 | BH I | 30 Beispiel 4 | 40 | 6,3 |
| 80 | BH I | 20 Beispiel 2 | 45 | 6,1 |
| 65 | BH II | 35 Beispiel 5 | 35 | 6,7 |
| 80 | BH III | 20 Beispiel 1 | 40 | 5,8 |
| 70 | BH III | 30 Beispiel 3 | 50 | 5,6 |
| 75 | BH IV | 25 Beispiel 6 | 45 | 6,0 |
| 80 | BH V | 20 Beispiel 2 | 55 | 6,2 |

Die Überzugsmittel werden gegebenenfalls nach Einarbeitung der Pigmente und Füllstoffe und/oder Katalysatoren gegebenenfalls unter Mitverwendung von Hilfslösungsmitteln mit Wasser auf die Applikationsviskosität verdünnt. Die bei 160 bis 180 °C eingebrannten Filme ergeben hinsichtlich ihrer mechanischen Eigenschaften sowie ihrer Widerstandsfähigkeit gegenüber korrosiven Medien ausgezeichnete Prüfergebnisse. Insbesonders bei der kathodischen Abscheidung nach dem ETL-Verfahren, lassen sich Grundierungen mit besonders günstigen Eigenschaften herstellen.

**Patentansprüche**

1. Verfahren zur Herstellung von Vernetzungskomponenten für durch Umesterung vernetzbare, wasserverdünnbare kationische Lackbindemittel, dadurch gekennzeichnet, daß man

(A) ein Knoevenagel-Reaktionsprodukt aus einer Carbonylverbindung und einer Verbindung der Formel

$$X{-}CH_2{-}Y$$

wobei
X —COOR, —CN oder —$COCH_3$,
Y —COOR ist und
R einen Alkylrest mit 1 bis 4 C-Atomen darstellt,
bei 50 bis 100 °C mit

(B) einem eine sekundäre Aminogruppe aufweisenden substituierten Oxazolidin der allgemeinen Formel

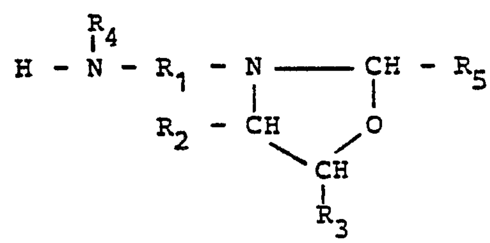

wobei
$R_1$ ein geradkettiger oder verzweigter oder cyclischer Alkylenrest mit 2 bis 12 C-Atomen oder ein Aralkylrest,
$R_2$ ein Wasserstoffatom oder eine Methylgruppe,
$R_3$ ein Wasserstoffatom oder ein Alkylrest,
$R_4$ ein nach Reaktion mit einem aktiven Wasserstoffatom verbleibender Rest eines in bezug auf die Doppelbindungen monofunktionellen Acryl- oder Methacrylmonomeren und
$R_5$ ein Wasserstoffatom oder ein Alkylrest mit 1 bis 12 C-Atomen oder ein gegebenenfalls substituierter Arylrest ist,
im Sinne einer Michael-Addition reagiert und das Reaktionsprodukt über die aktive Methylengruppe mit

(C) einem Di- oder Polyisocyanat umsetzt, wobei die Menge an Isocyanatgruppen den aktiven Methylengruppen der Oxazolidinringe entspricht.

2. Verfahren nach Anspruch 1, dadurch gekennzeichnet, daß man als Verbindungen der Formel $X{-}CH_2{-}Y$ Diester der Malonsäure mit $C_1$-$C_4$-Alkanolen einsetzt.

3. Verfahren nach Anspruch 1, dadurch gekennzeichnet, daß man als Verbindungen der Formel X—$CH_2$—Y Alkyl- oder Cycloalkylester der Cyanessigsäure oder die Methyl- oder Äthylester der Acetessigsäure einsetzt.

4. Verfahren nach den Ansprüchen 1 bis 3, dadurch gekennzeichnet, daß man als Carbonylverbindung zur Herstellung der Komponente (A) Aldehyde, vorzugsweise Formaldehyd einsetzt.

5. Verfahren nach den Ansprüchen 1 bis 4, dadurch gekennzeichnet, daß man die Knoevenagel-Reaktion zur Bildung der Komponente (A) und die Michael-Addition mit der Oxazolidinverbindung (B) in einer gemeinsamen Reaktion durchführt und das Reaktionsprodukt mit der Komponente (C) umsetzt.

6. Verfahren nach den Ansprüchen 1 bis 5, dadurch gekennzeichnet, daß man die Knoevenagel-Reaktion in der Variante nach Cope unter azeotroper Abtrennung des Reaktionswassers führt.

7. Verwendung der gemäß den Ansprüchen 1 bis 6 hergestellten Vernetzungskomponenten in Kombination mit wasserverdünnbaren, hydroxyl- und/oder aminofunktionellen Kunstharzen für wasserverdünnbare Einbrennlacke.

8. Verwendung der gemäß den Ansprüchen 1 bis 6 hergestellten Vernetzungskomponenten in Kombination mit wasserverdünnbaren, hydroxyl- und/oder aminofunktionellen Kunstharzen zur Formulierung von kathodisch abscheidbaren Elektrotauchlacken.

**Claims**

1. Process for producing crosslinking components for water dilutable cationic paint binders, crosslinkable through transesterification, characterised in that

(A) a Knoevenagel-reaction product of a carbonyl compound and a compound of the formula

$$X—CH_2—Y$$

wherein
X is —COOR, —CN or —$COCH_3$
Y is —COOR and
R is an alkyl radical with from 1 to 4 carbon atoms,
is reacted at 50 to 100 °C in the sense of a Michael-reaction with
(B) a substituted oxazolidine, carrying a secondary amino group, of the general formula

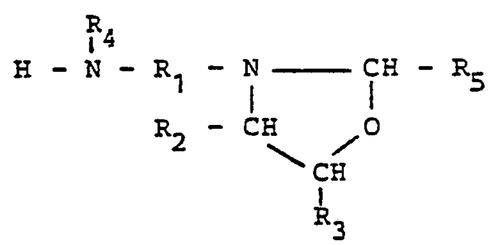

wherein
$R_1$ is a straight chain or branched or cyclic alkylene radical with from 2 to 12 carbon atoms or an aralkyl radical,
$R_2$ is a hydrogen atom or a methyl group,
$R_3$ is a hydrogen atom or an alkyl radical,
$R_4$ is a radical of an acrylic or methacrylic monomer, remaining after the reaction with an active hydrogen atom, the monomers being monofunctional with regard to the double bond,
$R_5$ is a hydrogen atom or an alkyl radical with from 1 to 12 carbon atoms or an optionally substituted aryl radical,
and the reaction product is reacted *via* the active methylene group with
(C) a di- or polyisocyanate, the quantity of isocyanate groups corresponding to the active methylene groups of the oxazolidine rings.

2. Process according to claim 1, characterised in that as compounds of formula X—$CH_2$—Y diesters of malonic acid with $C_1$-$C_4$-alkanols are used.

3. Process according to claim 1, characterised in that as compounds of formula X—$CH_2$—Y alkyl- or cycloalkylesters of cyanoacetic acid or the methyl- or ethylesters of acetoacetic acid are used.

4. Process according to claims 1 to 3, characterised in that as the carbonyl compounds for producing component (A) aldehydes, preferably formaldehyde, are used.

5. Process according to claims 1 to 4, characterised in that the Knoevenhagel-reaction for the formation of component (A) and the Michael-addition with the oxazolidine compound (B) is carried out in a simultaneous reaction and that the reaction product is reacted with component (C).

6. Process according to claims 1 to 5, characterised in that the Knoevenagel-reaction is carried out in the alternative according to Cope with azeotropic separation of the reaction water.

7. Use of the crosslinking components according to claims 1 to 6 in combination with water dilutable hydroxy- and/or amino-functional synthetic resins for water dilutable stoving paints.

8. Use of the crosslinking components according to claims 1 to 6 in combination with water dilutable, hydroxy- and/or amino-functional synthetic resins for the formulation of cathodically depositable electrodeposition paints.

**Revendications**

1. Procédé pour la préparation de composants de réticulation pour des liants de peintures cationiques diluables dans l'eau, réticulables par transestérification, caractérisé en ce que l'on fait réagir

(A) un produit de réaction de Knoevenagel obtenu à partir d'un composé carbonyle et d'un composé de formule

$$X{-}CH_2{-}Y$$

dans laquelle,
X représente $-COOR$, $-CN$ ou $-COCH_3$,
Y représente $-COOR$ et
R représente un radical alcoyle ayant de 1 à 4 atomes de C,
à une température vers 50 à 100 °C avec

(B) une oxazolidine substituée, présentant un groupe amino secondaire de formule générale

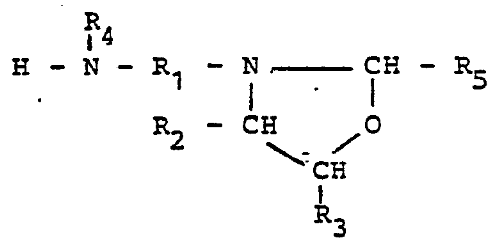

dans laquelle,
$R_1$ représente un radical alcoylène linéaire ou ramifié ou cyclique ayant 2 à 12 atomes de C ou un radical aralcoylène,
$R_2$ représente un atome d'hydrogène ou un groupe méthyle,
$R_3$ représente un atome d'hydrogène ou un radical alcoyle,
$R_4$ représente un radical d'un monomère acrylique ou méthacrylique, monofonctionnel par rapport aux liaisons doubles, subsistant après réaction avec un atome d'hydrogène actif, et
$R_5$ représente un atome d'hydrogène ou un radical alcoyle ayant 1 à 12 atomes de C ou un radical aryle éventuellement substitué,
la réaction s'effectuant au sens d'une addition de Michael, le produit réactionnel étant mis en réaction par l'intermédiaire des groupes méthylène actifs avec

(C) un di- ou polyisocyanate, la quantité en groupes isocyanates correspondant aux groupes méthylène actifs des cycles oxazolidine.

2. Procédé selon la revendication 1, caractérisé en ce que l'on met en œuvre en tant que composés de formule $X{-}CH_2{-}Y$ des diesters de l'acide malonique avec des alcanols en $C_1$-$C_4$.

3. Procédé selon la revendication 1, caractérisé en ce que l'on met en œuvre en tant que composés de formule $X{-}CH_2{-}Y$, des esters alcoylés ou cycloalcoylés de l'acide cyanacétique ou les esters méthyle ou éthyle de l'acide acétoacétique.

4. Procédé selon les revendications 1 à 3, caractérisé en ce que l'on met en œuvre en tant que composé carbonyle pour la préparation du composant (A), des aldéhydes, de préférence du formaldéhyde.

5. Procédé selon les revendications 1 à 4, caractérisé en ce que la réaction de Knoevenagel pour la formation du composant (A) et l'addition de Michael avec le composé oxazolidine (B) sont effectuées dans le cadre d'une réaction commune et que le produit de la réaction est mis à réagir avec le composant (C).

6. Procédé selon les revendications 1 à 5, caractérisé en ce que l'on effectue la réaction de Knoevenagel selon la variante d'après Cope sous séparation azéotrope de l'eau de réaction.

7. Utilisation des composants de réticulation préparés selon les revendications 1 à 6 en combinaison avec des résines synthétiques à fonction hydroxyle et/ou amino, diluables dans l'eau, pour des peintures de cuisson diluables dans l'eau.

8. Utilisation des composants de réticulation préparés selon les revendications 1 à 6, en combinaison avec des résines synthétiques à fonction hydroxyle et/ou amino, diluables dans l'eau, pour la formulation de peintures électrophorétiques, déposables cathodiquement.